# EUROPÄISCHE PATENTSCHRIFT

(11) **EP 3 979 981 B1**
(45) Veröffentlichungstag und Bekanntmachung des Hinweises auf die Patenterteilung: **08.07.2026**
(21) Anmeldenummer: 21725961.3
(22) Anmeldetag: 11.05.2021
(51) Int. Cl.: A61K 36/534, A61P 11/04, A61Q 11/00, A61K 9/00, A61K 9/10, A61K 31/23, A61K 31/355, A61K 31/4188, A61K 31/525, A61K 31/593, A61K 31/714, A61K 36/53, A61K 36/61, A61K 36/9068

(54) **SPRÜHFÄHIGE ZUSAMMENSETZUNG EINES RACHENSPRAYS**
SPRAYABLE COMPOSITION OF A THROAT SPRAY
COMPOSITION PULVÉRISABLE D'UN SPRAY POUR LA GORGE

(30) Priorität: 27.08.2020 DE 102020122469; 27.08.2020 DE 202020104968 U
(43) Veröffentlichungstag der Anmeldung: 13.04.2022
(60) Teilanmeldung: 23159462.3
(73) Patentinhaber: PM-International AG, 5445 Schengen (LU)
(72) Erfinder: SORG, Rolf, 5444 Schengen (LU); KÜHNE, Tobias, 54497 Morbach (DE); WAJDA, Rudi, 68259 Mannheim (DE); MEINHARDT, Horst, 56249 Herschbach (DE)
(74) Vertreter: Meissner Bolte Partnerschaft mbB
(86) Internationale Anmeldenummer: PCT/EP2021/062442
(87) Internationale Veröffentlichungsnummer: WO 2022/042893

(56) Entgegenhaltungen:
- WO-A1-2019/233552
- WO-A1-2020/143910
- WO-A2-02/43719

## Beschreibung

Die Erfindung betrifft eine Zusammensetzung, ein Spray, das die erfindungsgemäße Zusammensetzung enthält, die Verwendung einer Sprühvorrichtung zum Verteilen einer erfindungsgemäßen Zusammensetzung und ein nichtmedizinisches Verfahren zur Konditionierung eines Abschnittes des menschlichen Körpers.

Flüssige Zusammensetzungen zur Anwendung in der Mundhöhle und dem Rachen sind bekannt. Diese Zusammensetzungen werden u.a. als Spray unter den synonymen Bezeichnungen "Rachenspray", "Mundspray" oder "Halsspray" vertrieben. Bei diesen Zusammensetzungen bzw. Sprays kann es sich entweder um Nahrungsmittel, Nahrungsergänzungsmittel oder medizinische Produkte handeln.

Beispielsweise existieren diverse Sprays, die flüssige Zusammensetzungen mit desinfizierenden Wirkstoffen, wie z.B. Cetylpyridinium, Dequalinium, Hexamidin, und/oder Lokalanästhetika, wie z.B. Lidocain, und/oder nichtsteroidalen Antirheumatika, wie z.B. Flurbiprofen, enthalten. Manche Zusammensetzungen in den Sprays enthalten pflanzliche Öle, z.B. Kamillenöl, oder andere fettlösliche Verbindungen wie Zimtaldehyd oder Levomenthol. Manche Zusammensetzungen in den Sprays enthalten Vitamine und/oder Aminosäuren und/oder Mineralien.

Die WO 02/43719 A2 offenbart eine Zusammensetzung und ein Verfahren, bei denen eine Lösung gegen Schnarchen, die mindestens ein Homopolysaccharid enthält, an die Rachenschleimhäute, z.B. an den weichen Gaumen und das Zäpfchen, verabreicht wird. Die Lösung enthält vorzugsweise Beta-Glucan aus Hafer und ein geeignetes Verabreichungsmittel, das die Wirkstoffe in Lösung hält, und kann gegebenenfalls mit ätherischen Ölverbindungen, Vitaminen und/oder Aromastoffen kombiniert werden. Die Lösung wird vorzugsweise in Form eines Rachensprays verabreicht.

Neben den bereits genannten Sprays zur Anwendung in der Mundhöhle und dem Rachen existieren auch Hautsprays, die ähnliche Zusammensetzungen mit Wirkstoffen wie z.B. Hydrocortison enthalten.

Die bekannten Zusammensetzungen weisen in der Regel eine wässrige Grundlage auf. Die Anwendung dieser Zusammensetzungen erfordert daher, dass die Zusammensetzungen mikrobiologisch stabil sind, d.h. dass das Wachstum von Bakterien- oder Pilzkulturen in den Zusammensetzungen unterbleibt. Um die mikrobiologische Stabilität der Zusammensetzungen auch nach Anbruch zu gewährleisten, ist in der Regel die Zugabe von Konservierungsmitteln erforderlich.

Zur Konservierung werden meist künstliche Konservierungsmittel, wie z.B. Sorbinsäure und Derivate davon, Parabene (Derivate der 4-Hydroxybenzoesäure, insbesondere 4-Hydroxybenzoesäuremethylester und 4-Hydroxybenzoesäurepropylester), Natriumedetat und Glycerintriacetat, beigesetzt. Manche dieser Verbindungen sind unter anderem wegen möglicher Unverträglichkeiten umstritten. Da Konservierungsmittel in der Regel lipophil sind, gehen diese in Zusammensetzungen mit wässriger und lipophiler Phase außerdem zumindest teilweise in die lipophile Phase über. Damit die Konzentration an Konservierungsmittel in der wässrigen Phase ausreichend hoch ist, um eine Konservierung zu bewirken, werden zweiphasige Systeme daher in der Regel mit einem hohen Gehalt an Konservierungsmitteln formuliert.

Die bekannten Zusammensetzungen enthalten meist wasserlösliche und fettlösliche Verbindungen. Daher ist in der Regel auch die Zugabe von Löslichkeitsvermittlern erforderlich, wie z.B. Ethanol, Propylenglycol und Derivate davon, Macrogol und Derivate davon, Cellulose und Derivate davon (z.B. Hypromellose), Cyclodextrin und Derivate davon, anionische, kationische und nichtionische Tenside. Allerdings können Tenside die Wirksamkeit der Konservierungsmittel beeinträchtigen. Ein Produkt mit Ethanol steht außerdem nicht jedem zur Verfügung, Schwangere, Kinder und Personen mit Suchthistorie sind ausgeschlossen. Auch andere Lösevermittler sind zumindest umstritten, insbesondere bei oraler Aufnahme, z.B. Propylenglycol und Natriumdodecylsulfat wegen Hautreizungen, Macrogolglycerolricinoleat wegen Magenverstimmungen, Durchfall sowie Haut- und Schleimhautreizungen. Es besteht auch das Problem, das zweiphasige Zusammensetzungen, also Mischungen, die eine wässrige Phase und eine Lipidphase aufweisen, nicht dauerhaft physikalisch stabil sind und eine Zeit nach der Herstellung eine Phasentrennung auftritt.

Manche Verbindungen werden eingesetzt, weil sie sowohl konservierende als auch solubilisierende Eigenschaften aufweisen, z.B. Tetraalkylammoniumverbindungen, wie Benzalkonium, Benzethonium, Cetylalkonium, Cetylpyridinium, Cetyltrimethylammonium, Dequalinium, und die Alkohole Ethanol und Propylenglycol.

Zur Verbesserung des Geschmackes werden den Zusammensetzungen häufig auch künstliche Süßstoffe, Monosaccharide oder Disaccharide beigemischt.

Auch konzentrierte, wässrige Lösungen des Disaccharides Sucrose werden als Grundlage für Sprays eingesetzt, weil die Sucrose in hoher Konzentration zur Konservierung und als Geschmacksträger geeignet ist. Nachteilig ist natürlich, dass eine solche Zusammensetzung kariogen ist und außerdem Blutzuckerspitzen verursacht, was insbesondere für Diabetiker nachteilig ist. Gerade Diabetiker sind aber manchen Risiken besonders ausgesetzt, so dass gerade für Diabetiker möglichst jedes Produkt, das einen ernährungsphysiologischen, gesundheitlichen oder krankheitsvorbeugenden Nutzen aufweist, geeignet sein sollte.

Ein Hindernis bei der Verwendung flüssiger Zusammensetzungen in Sprays ist, dass die Zusammensetzungen über eine Sprühvorrichtung sprühbar sein müssen. Zusammensetzungen mit hoher Viskosität, z.B. Öle, oder Zusammensetzungen, die Feststoffe enthalten, sind oft nicht sprühbar oder können höchstens in Form eines starken Strahles und ohne Zerstäubung appliziert werden.

Vor diesem Hintergrund besteht eine Aufgabe der Erfindung darin, eine Zusammensetzung bereitzustellen, die zum Besprühen eines Abschnittes des menschlichen Körpers, insbesondere der Mundhöhle und des Rachens, geeignet ist und gegenüber den Zusammensetzungen bekannter Rachensprays Vorteile aufweist. Die Zusammensetzung soll mikrobiologisch stabil sein und sowohl wasserlösliche als auch fettlösliche Verbindungen sollten enthalten sein bzw. in der Zusammensetzung lösbar oder zumindest dispergierbar sein.

Aus den Bestrebungen, diese Aufgabe zu lösen, resultiert eine Zusammensetzung, die dadurch gekennzeichnet ist, dass die Zusammensetzung aus einer polaren Phase und einer unpolaren Phase besteht, wobei
die polare Phase bezogen auf das Gesamtgewicht der polaren Phase mindestens 40 Gew.-% Glycerin und mindestens 20 Gew.-% Wasser enthält, die Zusammensetzung pro 100 g der Zusammensetzung 0,5 g bis 4 g Lecithin enthält, und die unpolare Phase bzw. die Zusammensetzung mindestens einen Extrakt enthält, der aus einem Extrakt aus der Minze (Mentha arvensis oder Mentha x piperita), einem Extrakt aus dem Gewürznelkenbaum (Syzygium aromaticum), einem Extrakt aus dem Ingwer (Zingiber officinale) und einem Extrakt aus dem Echten Thymian (Thymus vulgaris) ausgewählt ist, wobei die polare Phase bezogen auf das Gesamtgewicht der polaren Phase 53 Gew.-% bis 78 Gew.-% Glycerin enthält und die unpolare Phase pro 100 g der Zusammensetzung 0,4 g bis 10,0 g des Extraktes oder der Extrakte insgesamt enthält.

Die flüssige Zusammensetzung besteht aus einer polaren, wasserhaltigen Phase und einer unpolaren Phase, enthaltend unpolare Verbindungen, und bildet doch - zumindest bei Beobachtung mit dem Auge - eine homogene Mischung. Mit anderen Worten, die Zusammensetzung enthält wasserlösliche und fettlösliche Verbindungen, wobei die Verbindungen gelöst oder zumindest dispergiert sind. Die Zusammensetzung ist also physikalisch stabil. Hierzu trägt die spezielle Grundlage aus Glycerin, Wasser und Lecithin in bestimmten Mengenverhältnissen bei. Die Zusammensetzung ist zudem mikrobiologisch stabil. Zu der mikrobiologischen Stabilität trägt insbesondere der Glyceringehalt und der mindestens eine Extrakt bei. Der mindestens eine Extrakt kann ätherisches Öl enthalten. Nur mittels natürlich vorkommender Inhaltsstoffe wird also eine mikrobiologisch und physikalisch stabile Zusammensetzung erhalten, die zur oralen Applikation geeignet ist. Dadurch wird die Zusammensetzung physiologisch und psychologisch besonders positiv angenommen und ist besonders gut verträglich. Die Zusammensetzung weist ein geringes Risiko für Unverträglichkeiten wie Allergien auf. In der Zusammensetzung lösen sich sowohl wasserlösliche als auch fettlösliche Verbindungen und können enthalten sein, ohne dass zusätzliche Lösevermittler oder Tenside erforderlich sind. Somit erlaubt die Grundlage der Zusammensetzung, in die Zusammensetzung hydrophile und hydrophobe Verbindungen einzuarbeiten, um die Eigenschaften der Zusammensetzung weiter zu modifizieren oder um die Zusammensetzung als Trägersystem für bestimmte Verbindungen zu nutzen.

Synthetische oder nicht natürlich vorkommende Zusatzstoffe zur Konservierung oder Solubilisierung sind nicht erforderlich. Die Zusammensetzung weist auch ohne Monosaccharide und Disaccharide einen guten Geschmack und Geruch auf und ist dadurch nicht kariogen und verursacht keine Blutzuckerspitzen. Die Zusammensetzung ist vor allem sprühbar. Trotz des hohen Anteils an relativ viskosem Glycerin und den/dem öligen Extrakt(en) kann die Zusammensetzung in Form eines Sprays appliziert werden. Die spezielle Grundlage ermöglicht auch, dass eine lange Wechselwirkung mit einem besprühten Körperteil und ein Austausch zwischen Zusammensetzung und besprühtem Körperteil erfolgen kann. Durch den mindestens einen Extrakt werden neben der Konservierung eine Vielzahl positiver Effekte erreicht. Die Inhaltsstoffe der Extrakte können an der Applikationsstelle wirken, den Geschmack, den Atemgeruch (Mundgeruch) und das Mundgefühl verbessern und gegebenenfalls sogar antibakteriell und/oder antiviral wirken.

Bei dem Extrakt aus der Minze kann es sich um einen Extrakt aus der Ackerminze (Mentha arvensis) oder aus der Pfefferminze (Mentha x piperita) handeln. Im Rahmen der Erfindung ist ein Extrakt aus der Ackerminze (Mentha arvensis) bevorzugt.

Bei dem Extrakt aus der Ackerminze (Mentha arvensis) handelt es sich bevorzugt um einen Extrakt aus den oberirdischen Teilen, vorzugsweise den blühenden, oberirdischen Teilen. Dabei kann es sich um Minzöl gemäß Europäischem Arzneibuch handeln. Der Extrakt kann z.B. durch Wasserdampfextraktion gewonnen sein. Die Inhaltsstoffe des Extraktes können antioxidativ, möglicherweise auch antibakteriell und/oder antiviral, wirken und einen wohlriechenden Geschmack und Atem vermitteln.

In einer anderen Ausführungsform der Erfindung wird statt dem Extrakt aus der Ackerminze (Mentha arvensis) ein Extrakt aus der Pfefferminze (Mentha x piperita) verwendet. Bei dem Extrakt aus der Pfefferminze handelt es sich bevorzugt um einen Extrakt aus den oberirdischen Teilen, vorzugsweise den blühenden, oberirdischen Teilen. Dabei kann es sich um Pfefferminzöl gemäß Europäischem Arzneibuch handeln. Der Extrakt kann z.B. durch Wasserdampfextraktion gewonnen sein. Die Inhaltsstoffe des Extraktes können antioxidative und möglicherweise antibakterielle und/oder antivirale Wirkungen aufweisen und einen wohlriechenden Geschmack und Atem vermitteln.

Bei dem Extrakt aus dem Gewürznelkenbaum (Syzygium aromaticum) handelt es sich bevorzugt um einen Extrakt aus den Gewürznelken, vorzugsweise Gewürznelkenöl. Vorzugsweise wird der Extrakt aus den Blütenknospen, bevorzugter den getrockneten Blütenknospen, gewonnen. Dabei kann es sich um Gewürznelken gemäß Europäischem Arzneibuch handeln. Die Inhaltsstoffe des Extraktes können antioxidative Wirkungen aufweisen und einen wohlriechenden Geschmack und Atem vermitteln.

Bei dem Extrakt aus dem aus dem Ingwer (Zingiber officinale) handelt es sich bevorzugt um einen Extrakt aus Wurzelstöcken des Ingwers, vorzugsweise aus getrockneten Wurzelstöcken des Ingwers. Dabei kann es sich um Ingwerwurzelstock gemäß Europäischem Arzneibuch handeln. Vorzugsweise ist der Extrakt ein CO₂-Destraktionsextrakt. Die Inhaltsstoffe des Extraktes können antioxidative Wirkungen aufweisen.

Bei dem Extrakt aus dem aus dem Echten Thymian (Thymus vulgaris) handelt es sich bevorzugt um einen Extrakt aus den blühenden oberirdischen Teilen. Dabei kann es sich um Thymianöl gemäß Europäischem Arzneibuch handeln. Der Extrakt kann z.B. durch Wasserdampfextraktion gewonnen sein. Die Inhaltsstoffe des Extraktes können möglicherweise antibakterielle und antivirale Wirkungen aufweisen.

Lecithin ist eine Zubereitung, die Phosphatidylcholine als Hauptbestandteil enthält. Phosphatidylcholine sind 1,2-Diacyl-*sn*-glycero-3-phosphocholin, Phospholipide, in denen Phosphorsäure mit Diacylglycerol und Cholin verestert sind. Lecithin ist nach der Zusatzstoff-Zulassungsverordnung (ZZulV 1998) unter der Nummer E 322 ein für Lebensmittel allgemein (ausgenommen bestimmte Lebensmittel) zugelassener Zusatzstoff.

Die erfindungsgemäße Zusammensetzung enthält pro 100 g der Zusammensetzung 0,5 g bis 4 g, bevorzugter 1 g bis 3,5 g, noch bevorzugter 1,5 g bis 3,0 g oder 2,0 g bis 3,0 g Lecithin. Das Lecithin liegt überwiegend im Grenzbereich zwischen polarer und unpolarer Phase vor. Mit anderen Worten, die Zusammensetzung besteht neben der polaren Phase und der unpolaren Phase auch noch aus einer Lecithinphase, die aus dem Lecithin in der Zusammensetzung besteht. Das Lecithin weist einen Gehalt an Phosphatidylcholin von 30 Gew.-% bis 100 Gew.-% auf. Mit anderen Worten, die Zusammensetzung enthält vorzugsweise pro 100 g der Zusammensetzung 0,2 g bis 4 g, bevorzugter 0,3 g bis 3,5 g, noch bevorzugter 0,5 g bis 3,0 g oder 0,6 g bis 3,0 g, Phosphatidylcholin. In einer Ausführungsform enthält die erfindungsgemäße Zusammensetzung pro 100 g der Zusammensetzung 1 g bis 3 g, bevorzugt 1 g bis 2 g, Phosphatidylcholin. Als Lecithin werden vorzugsweise entölte Lecithinfraktionen eingesetzt. Dies bedeutet, dass das Lecithin vorzugsweise im Wesentlichen frei von unpolaren Lipiden wie Fettsäuren und Triglyceriden ist. Der Gehalt an unpolaren Lipiden wie Fettsäuren und Triglyceriden beträgt bevorzugt weniger als 3 Gew.-%. Das Lecithin enthält einen Anteil an polaren Lipiden (acetonunlöslicher Substanz) von 90 bis 100 Gew.-%. Das Lecithin kann aus einer Quelle ausgewählt aus Soja, Sonnenblume, Raps, Fisch, Milch und/oder Eiern stammen, wobei nichttierische Quellen wie Soja, Sonnenblume und Raps besonders bevorzugt sind. Neben dem Hauptbestandteil Phosphatidylcholin kann Lecithin je nach Herkunft Phosphatidylethanolamin, Phosphatidylinositol, Phosphatidylserin, Sterole, Fette und Öle enthalten. Beispielsweise kann ein Lecithin aus Soja zu 40-50 Gew.-% Phosphatidylcholin, zu ca. 10 Gew.-% Phosphatidylethanolamin, zu ca. 5 Gew.-% Phosphatidylinositol, zu ca. 1-2 Gew.-% Phosphatidylserin und ferner Sterole, Fette und Öle enthalten. Aus Eidottern erhaltenes Lecithin besteht dagegen im Wesentlichen aus Phosphatidylcholin.

Wasser und Glycerin, gegebenenfalls auch Polysaccharide und wasserlösliche Vitamine, sind Bestandteile der polaren Phase.

Die unpolare Phase kann aus Fettsäuren und deren Estern, insbesondere Triglyceriden (Triacylglycerolen) und Ethylestern, fettlöslichen Vitaminen sowie Carotinoiden, wie z.B. Lutein und Zeaxanthin, Vitamin A (Retinol) und dessen Derivaten Retinal, Retinylpalmitat und Retinylacetat, Vitamin E als Sammelbegriff für Tocopherole und Tocotrienole (darunter fallen alpha-, beta-, gamma-, delta-Tocopherole und Tocotrienole, sowohl als natürliche Gemische als auch synthetisch in Reinform), Vitamin E-Derivaten wie alpha-, beta-, gamma- und delta-Tocopherolacetat (sowohl in optisch reiner Form als auch als Racemat), Vitamin D2 (Ergocalciferol), Vitamin D3 (Cholecalciferol), Vitamin K1 (Phyllochinon), Vitamin K2 (Menachinon), Vitamin K3 (Menadion), Coenzym Q 10 H (Ubichinol), Ubichinon-10 und Perfluorcarbonen gebildet werden. In der vorliegenden Erfindung kommen als Quelle für die Bestandteile der unpolaren Phase insbesondere der Extrakt bzw. die Extrakte aus Minze (Mentha arvensis oder Mentha x piperita), insbesondere Minzöl und/oder Pfefferminzöl, aus dem Gewürznelkenbaum (Syzygium aromaticum), insbesondere Gewürznelkenöl, aus dem Ingwer (Zingiber officinale) und aus dem Echten Thymian (Thymus vulgaris), insbesondere Thymianöl, sowie Kokosöl und fettlösliche Vitamine in Frage.

Bei der Zusammensetzung bzw. dem Spray handelt es sich um ein Nahrungsergänzungsmittel oder um ein Nahrungsmittel.

Die erfindungsgemäße Zusammensetzung ist bei Raumtemperatur (25 °C) flüssig bzw. eine Flüssigkeit.

Ausführungsformen innerhalb dieser Schrift können beliebig miteinander kombiniert werden, sofern sich aus dem Gegenstand und der Beschreibung der Ausführungsformen nicht eindeutig Gegenteiliges ergibt.

Die Verben "enthalten" und "umfassen" und ihre Konjugationen enthalten auch das Verb "bestehen aus" mit seinen Konjugationen.

Bevorzugte Ausführungsformen sind auch in den Patentansprüchen wiedergegeben.

Die polare Phase der erfindungsgemäßen Zusammensetzung enthält mindestens 40 Gew.-% Glycerin, bezogen auf das Gesamtgewicht der polaren Phase. Ein hoher Gehalt an Glycerin unterstützt die mikrobiologische Konservierung der Zusammensetzung. Die Erfinder haben entdeckt, dass durch den Einsatz des Extraktes bzw. der Extrakte der Anteil an konservierendem Glycerin an der polaren Phase und der Zusammensetzung gesenkt werden kann und der Anteil an Wasser erhöht werden kann. Hierdurch wird die Zusammensetzung sprühbar. Sprühbar im Sinne der Erfindung bedeutet, dass die Zusammensetzung in Form von Tröpfchen oder eines zerstäubenden Sprühstrahles abgegeben werden kann, z.B. aus einer für Rachensprays üblichen Sprühvorrichtung. Mit anderen Worten, die Zusammensetzung kann mit einer für Rachensprays üblichen Sprühvorrichtung (Zerstäuber) zerstäubt werden. Nicht sprühbar im Sinne der Erfindung sind Zusammensetzungen, die von üblichen Sprühvorrichtungen für Rachensprays nicht oder in Form eines starken Strahles abgegeben werden.

Vorzugsweise ist die Zusammensetzung nicht nur sprühbar und geeignet, vom Menschen oral eingenommen zu werden, sondern auch geeignet, in den menschlichen Rachen gesprüht zu werden.

Vorzugsweise enthält die unpolare Phase (und die Zusammensetzung) einen Extrakt aus der Minze (Mentha arvensis oder Mentha x piperita), einen Extrakt aus dem Gewürznelkenbaum (Syzygium aromaticum), einen Extrakt aus dem Ingwer (Zingiber officinale) und einen Extrakt aus dem Echten Thymian (Thymus vulgaris). Durch die Kombination dieser Extrakte werden die oben beschriebenen Effekte besonders gut ausgenutzt.

Bevorzugter enthält die unpolare Phase (und die Zusammensetzung) einen Extrakt aus der Ackerminze (Mentha arvensis), einen Extrakt aus dem Gewürznelkenbaum (Syzygium aromaticum), einen Extrakt aus dem Ingwer (Zingiber officinale) und einen Extrakt aus dem Echten Thymian (Thymus vulgaris). Durch die Kombination dieser Extrakte werden die oben beschriebenen Effekte besonders gut ausgenutzt.

Vorzugsweise enthält die unpolare Phase (und die Zusammensetzung) pro 100 g der Zusammensetzung 1,5 g bis 7,0 g des Extraktes oder der Extrakte insgesamt.

Vorzugsweise beträgt der Anteil der unpolaren Phase an der Zusammensetzung höchstens 25 Gew.-%, bevorzugter höchstens 20 Gew.-%, noch bevorzugter höchstens 15 Gew.-%, wobei die polare Phase und das Lecithin die Differenz ad 100 Gew.-% bilden. In einer bevorzugten Ausführungsform beträgt der Anteil der unpolaren Phase an der Zusammensetzung bezogen auf das Gesamtgewicht der Zusammensetzung 0,4 Gew.-% bis 10,0 Gew.-%, vorzugsweise 1,5 Gew.-% bis 7,0 Gew.-%, wobei die polare Phase und das Lecithin die Differenz ad 100 Gew.-% bilden. In einer bevorzugteren Ausführungsform beträgt der Anteil der unpolaren Phase an der Zusammensetzung 0,9 Gew.-% bis 15 Gew.-%, vorzugsweise 2,0 Gew.-% bis 12,0 Gew.-%, wobei die polare Phase und das Lecithin die Differenz ad 100 Gew.-% bilden.

Vorzugsweise enthält die polare Phase bezogen auf das Gesamtgewicht der polaren Phase 53 Gew.-% bis 67 Gew.-%, am bevorzugtesten 56 Gew.-% bis 64 Gew.-%, Glycerin.

Vorzugsweise enthält die Zusammensetzung (und die polare Phase) pro 100 g der Zusammensetzung 0,01 g bis 2,0 g Polysaccharide. Die Erfinder haben herausgefunden, dass Polysaccharide in dieser Menge einen positiven Effekt auf die Stabilität der Zusammensetzung und die Sprühfähigkeit haben. Vorzugsweise sind die Polysaccharide β-Glucane, besonders bevorzugt (1,3)-(1,6)-Beta-D-Glucane. β-Glucane sind aus β-D-Glucose-Einheiten (β-D-Glucopyranose) aufgebaute Polysaccharide.

Vorzugsweise enthält die unpolare Phase (und die Zusammensetzung) pro 100 g der Zusammensetzung 0,1 g bis 4,0 g, bevorzugt 1,0 g bis 2,5 g, eines Extraktes aus der Ackerminze (Mentha arvensis).

Vorzugsweise enthält die unpolare Phase (und die Zusammensetzung) pro 100 g der Zusammensetzung 0,1 g bis 2,0 g, bevorzugt 0,2 g bis 1,2 g, eines Extraktes aus dem Gewürznelkenbaum (Syzygium aromaticum).

Vorzugsweise enthält die unpolare Phase (und die Zusammensetzung) pro 100 g der Zusammensetzung 0,1 g bis 2,0 g, bevorzugt 0,2 g bis 0,8 g, eines Extraktes aus dem Ingwer (Zingiber officinale).

Vorzugsweise enthält die unpolare Phase (und die Zusammensetzung) pro 100 g der Zusammensetzung 0,1 g bis 2,0 g, bevorzugt 0,4 g bis 1,5 g, eines Extraktes aus dem Echten Thymian (Thymus vulgaris).

Besonders bevorzugt enthält die unpolare Phase (und die Zusammensetzung) pro 100 g der Zusammensetzung 0,1 g bis 4,0 g, bevorzugt 1,0 g bis 2,5 g, eines Extraktes aus der Ackerminze (Mentha arvensis), 0,1 g bis 2,0 g, bevorzugt 0,2 g bis 1,2 g, eines Extraktes aus dem Gewürznelkenbaum (Syzygium aromaticum), 0,1 g bis 2,0 g, bevorzugt 0,2 g bis 0,8 g, eines Extraktes aus dem Ingwer (Zingiber officinale) und 0,1 g bis 2,0 g, bevorzugt 0,4 g bis 1,5 g, eines Extraktes aus dem Echten Thymian (Thymus vulgaris).

Die Vorteile dieser Extrakte sind oben beschrieben. In diesen Konzentrationen sind die Extrakte besonders dienlich.

Vorzugsweise enthält die Zusammensetzung pro 100 g der Zusammensetzung 0,05 g bis 2,0 g, bevorzugt 0,1 g bis 0,8 g, eines Extraktes aus Weintrauben (Vitis vinifera), vorzugsweise aus Weintraubenkernen. Ein solcher Extrakt enthält Tannine. Tannine sind Polyphenole, die antioxidative, adstringierende und enzyminhibierende Eigenschaften aufweisen können. Tannine können auch ein besonderes Mundgefühl vermitteln. Von bestimmten Tanninen wurde berichtet, dass sie das Andocken eines Virus an seine Wirtszelle verhindern können, wodurch möglicherweise die Gefahr einer Ansteckung durch Krankheitserreger, die eine lokale oder allgemeine Störung des Organismus zur Folge hat, reduziert werden kann. Möglicherweise kann die erfindungsgemäße Zusammensetzung daher die Abwehr von Krankheitserregern unterstützen. Aufgrund der Formulierung der Zusammensetzung haftet die Zusammensetzung relativ lange am Anwendungsort und kann einwirken. In der Formulierung als Spray kann die Zusammensetzung direkt und fein verteilt aufgetragen werden.

Vorzugsweise enthält die unpolare Phase (und die Zusammensetzung) pro 100 g der Zusammensetzung zusätzlich zu gegebenenfalls in dem Extrakt / den Extrakten enthaltenen Triacylglycerolen 0,5 g bis 5,0 g, bevorzugt 1 g bis 3,0 g, Triacylglycerole. Vorzugsweise handelt es sich um mittelkettige Triacylglycerole, z.B. aus Kokosöl. Triacylglycerole können als Träger für lipophile Wirkstoffe und ätherische Öle dienen. Durch ihren Einsatz kann die Formulierung stabilisiert werden. Ferner kann das Sprühverhalten der Zusammensetzung in positiver Weise beeinflusst werden. Ferner wird durch die Triacylglycerole ein über einen längeren Zeitraum am besprühten Körperteil (z.B. Rachen) haftender Ölfilm gebildet, durch den lipophile Verbindungen diffundieren und wirken können.

Vorzugsweise enthält die Zusammensetzung pro 100 g der Zusammensetzung mindestens ein Vitamin, ausgewählt aus Vitamin B2, Vitamin B7 (Biotin), Vitamin B12, Vitamin D3 sowie Derivaten davon, Vitamin A sowie Derivaten davon und Vitamin E sowie Derivaten davon. Bevorzugter enthält die Zusammensetzung Vitamin B2, Vitamin B7 (Biotin), Vitamin B12, Vitamin D3 oder ein Derivat davon, Vitamin A oder ein Derivat davon und Vitamin E oder ein Derivat davon. In einer noch bevorzugteren Ausführungsform enthält die Zusammensetzung pro 100 g der Zusammensetzung 0,1 g bis 0,3 g Vitamin B2, 0,004 g bis 0,01 g Vitamin B7 (Biotin), 0,0002 g bis 0,0006 g Vitamin B12, 10000 IE bis 50000 IE Vitamin D3, 0,05 g bis 0,2 g Retinylpalmitat und 1 g bis 3 g Vitamin E-Acetat. Vitamin A, Vitamin D3 und Vitamin B12 können zu einer normalen Funktion des Immunsystems beitragen. Vitamin B2, Vitamin B7 (Biotin) und Vitamin A können zur Erhaltung normaler Schleimhäute beitragen. Vitamin E kann dazu beitragen, die Zellen vor oxidativem Stress zu schützen. Durch diese Vitamine wird die Gesundheit allgemein, insbesondere die Gesundheit der Zellen des Mund- und Rachenraumes, gefördert. Diese Vitamine sind teilweise wasserlöslich, teilweise fettlöslich. Dank der speziellen Formulierung können diese Vitamine simultan direkt auf die Schleimhäute des Rachens aufgetragen werden.

In einer besonders bevorzugten Ausführungsform besteht die Zusammensetzung aus einer polaren Phase und einer unpolaren Phase,
wobei die polare Phase bezogen auf das Gesamtgewicht der polaren Phase mindestens 20 Gew.-% Wasser enthält,
wobei die polare Phase bezogen auf das Gesamtgewicht der polaren Phase 53 Gew.-% bis 67 Gew.-%, am bevorzugtesten 56 Gew.-% bis 64 Gew.-%, Glycerin enthält,
wobei die Zusammensetzung neben Wasser und Glycerin pro 100 g der Zusammensetzung enthält:
   0,5 g bis 4 g, bevorzugter 1 g bis 3,5 g, noch bevorzugter 1,5 g bis 3,0 g oder 2,0 g bis 3,0 g, Lecithin (0,2 g bis 4 g, bevorzugter 0,3 g bis 3,5 g, noch bevorzugter 0,5 g bis 3,0 g oder 0,6 g bis 3,0 g, Phosphatidylcholin oder 1 g bis 3 g, bevorzugt 1 g bis 2 g, Phosphatidylcholin),
   - 0,1 g bis 4,0 g, bevorzugt 1,0 g bis 2,5 g, eines Extraktes aus der Ackerminze (Mentha arvensis),
   - 0,1 g bis 2,0 g, bevorzugt 0,2 g bis 1,2 g, eines Extraktes aus dem Gewürznelkenbaum (Syzygium aromaticum),
   - 0,1 g bis 2,0 g, bevorzugt 0,2 g bis 0,8 g, eines Extraktes aus dem Ingwer (Zingiber officinale),
   - 0,1 g bis 2 g, bevorzugt 0,4 g bis 1,5 g, eines Extraktes aus dem Echten Thymian (Thymus vulgaris),
   - 0,5 g bis 5,0 g, bevorzugt 1,0 g bis 3,0 g, Triacylglycerole, vorzugsweise mittelkettige Triacylglycerole,
   - 0,01 g bis 2,0 g Polysaccharide, bevorzugt β-Glucane, besonders bevorzugt (1,3)-(1,6)-Beta-D-Glucane,
   - 0,05 g bis 2,0 g, bevorzugt 0,1 g bis 0,8 g, eines Extraktes aus Weintrauben (Vitis vinifera), vorzugsweise aus Weintraubenkernen,
   - Vitamin B2, vorzugsweise 0,1 g bis 0,3 g Vitamin B2,
   - Vitamin B7 (Biotin), vorzugsweise 0,004 g bis 0,01 g Vitamin B7 (Biotin),
   - Vitamin B12, vorzugsweise 0,0002 g bis 0,0006 g Vitamin B12,
   - Vitamin D3, vorzugsweise 10000 IE bis 50000 IE Vitamin D3,
   - Retinylpalmitat, vorzugsweise 0,05 g bis 0,2 g Retinylpalmitat, und
   - Vitamin E-Acetat, vorzugsweise 1 g bis 3 g, Vitamin E-Acetat.

In einer besonders bevorzugten Ausführungsform besteht die Zusammensetzung aus diesen Bestandteilen, wobei Wasser und Glycerin die Differenz ad 100 g der Zusammensetzung bilden.

Die Grundlage einer erfindungsgemäßen Zusammensetzung wird in einer bevorzugten Ausführungsform aus einer Zusammensetzung gebildet, die pro 100 g der Zusammensetzung enthält:
- 25 g bis 35 g Wasser,
- 0,5 g bis 4 g Lecithin,
- 1 g bis 2,5 g Minzöl (Mentha arvensis),
- 0,2 g bis 1,2 g Gewürznelkenöl (Syzygium aromaticum),
- 0,2 g bis 0,8 g Ingwerwurzelstock-CO₂-Destraktionsextrakt (Zingiber officinale),
- 0,4 g bis 1,5 g Thymianöl (Thymus vulgaris) und
mindestens 50 g Glycerin. Die Zusammensetzung kann gemäß der Offenbarung dieser Schrift modifiziert werden.

In einer ganz besonders bevorzugten Ausführungsform besteht die Zusammensetzung pro 100 g der Zusammensetzung aus
- 33 g Wasser,
- 2 g Lecithin (1,5 g Phosphatidylcholin),
- 1,6 g Minzöl (Mentha arvensis),
- 0,4 g Gewürznelkenöl (Syzygium aromaticum),
- 0,4 g Ingwerwurzelstock-CO₂-Destraktionsextrakt (Zingiber officinale),
- 0,8 g Thymianöl (Thymus vulgaris),
- 2 g mittelkettigen Triacylglycerolen,
- 0,05 g (1,3)-(1,6)-Beta-D-Glucanen,
- 0,16 g eines Extraktes aus Weintraubenkernen (Vitis vinifera),
- 0,2 g Vitamin B2,
- 0,007 g Vitamin B7 (Biotin),
- 0,0004 g Vitamin B12,
- 10000 IE bis 50000 IE Vitamin D3,
- 0,13 g Retinylpalmitat,
- Vitamin E-Acetat, vorzugsweise 2 g Vitamin E-Acetat, und
- Glycerin ad 100 g.

In einer bevorzugten Ausführungsform enthält die Zusammensetzung kein Monosaccharid und kein Disaccharid. In einer bevorzugten Ausführungsform enthält die Zusammensetzung pro 100 g der Zusammensetzung insgesamt höchstes 1 g an Monosacchariden und Disacchariden. Als Monosaccharid im Sinne dieser Schrift gilt z.B. Glucose oder Fructose. Als Disaccharid im Sinne dieser Schrift gilt z.B. Saccharose.

In einer bevorzugten Ausführungsform enthält die Zusammensetzung keinen einwertigen Alkohol. In einer bevorzugten Ausführungsform enthält die Zusammensetzung pro 100 g der Zusammensetzung insgesamt höchstes 1 g an einwertigen Alkoholen. Als einwertiger Alkohol im Sinne dieser Schrift gelten z.B. Ethanol und Propanol.

In einer bevorzugten Ausführungsform enthält die Zusammensetzung keinen zweiwertigen Alkohol. In einer bevorzugten Ausführungsform enthält die Zusammensetzung pro 100 g der Zusammensetzung insgesamt höchstes 1 g an zweiwertigen Alkoholen. Als zweiwertiger Alkohol im Sinne dieser Schrift gilt z.B. Propylenglycol.

In einer bevorzugten Ausführungsform enthält die Zusammensetzung kein synthetisches Tensid. Der Begriff "kein" ist so zu verstehen, dass die Konzentration der Verbindung so gering ist, dass keine nennenswerte Wirkung zu erwarten ist. Mit anderen Worten, die Konzentration liegt außerhalb der Größenordnung, in der die Verbindung normalerweise eingesetzt wird. Als synthetisches Tensid im Sinne der Erfindung gelten ethoxylierte Tenside, Macrogol und Derivate davon (z.B. Macrogolglycerolfettsäureester wie z.B. Macrogolglycerolricinoleat, Macrogolfettsäureester wie z.B. Macrogololeat, Macrogolfettalkoholether wie z.B. Polyoxyethylencetylether), Propylenglycol und Derivate davon, Polyoxypropylen-Polyoxyethylen-Blockpolymere, Cellulose und Derivate davon (z.B. Hypromellose), Benzalkonium, Benzethonium, Cetylalkonium, Cetylpyridinium, Cetyltrimethylammonium, Dequalinium, Natriumdodecylsulfat, Natriumcetylsulfat, Natriumstearylsulfat, Ethylenglycolmonostearat, Partialfettsäureester des Sorbitans (z.B. Sorbitanmonolaurat, Sorbitanmonopalmitat, Sorbitanmonostearat, Sorbitantristearat, Sorbitanmonooleat, Sorbitansesquioleat, Sorbitantrioleat, Polysorbate (Polyoxyethylensorbitanfettsäureester), Fettsäureester der Saccharose, Fettsäureester des Polyglycerols und D-α-Tocopheryl-1000-succinat.

In einer bevorzugten Ausführungsform enthält die Zusammensetzung kein künstliches Konservierungsmittel. Der Begriff "kein" ist so zu verstehen, dass die Konzentration der Verbindung so gering ist, dass keine nennenswerte Wirkung zu erwarten ist. Mit anderen Worten, die Konzentration liegt außerhalb der Größenordnung, in der die Verbindung normalerweise eingesetzt wird. Als künstliches Konservierungsmittel im Sinne der Erfindung gelten 4-Hydroxybenzoesäureester (z.B. 4-Hydroxybenzoesäuremethylester und 4-Hydroxybenzoesäurepropylester), Chlorocresol, Chlorobutanol, Sorbinsäure (E 200), Kaliumsorbat (E 202), Calciumsorbat (E 203), Natriumedetat, Glycerintriacetat, Benzalkonium, Benzethonium, Cetylalkonium, Cetylpyridinium, Cetyltrimethylammonium, Cetrimonium, Chlorhexidin, Dequalinium, organische Quecksilberverbindungen.

In einer bevorzugten Ausführungsform enthält die Zusammensetzung keinen künstlichen Süßstoff. Der Begriff "kein" ist so zu verstehen, dass die Konzentration der Verbindung so gering ist, dass keine nennenswerte Wirkung zu erwarten ist. Mit anderen Worten, die Konzentration liegt außerhalb der Größenordnung, in der die Verbindung normalerweise eingesetzt wird. Als künstlicher Süßstoff im Sinne der Erfindung gelten Acesulfam (E 950), Advantam (E 969), Aspartam (E 951), Aspartam-Acesulfam-Salz (E 962), Cyclamat (E 952), Neohesperidin (E 959), Neotam (E 961), Saccharin (E 954) und Sucralose (E 955). Steviolglycoside, z.B. Steviosid (E 960), gelten nicht als künstliche Süßstoffe im Sinne der Erfindung.

In einer bevorzugten Ausführungsform ist die Zusammensetzung zuckerfrei im Sinne der Verordnung (EG) Nr. 1924/2006 des Europäischen Parlaments und des Rates vom 20. Dezember 2006 über nährwert- und gesundheitsbezogene Angaben über Lebensmittel. Mit anderen Worten, in einer bevorzugten Ausführungsform enthält die Zusammensetzung nicht mehr als 0,5 g Zucker (Monosaccharide und Disaccharide) pro 100 g der Zusammensetzung.

In einer bestimmten Ausführungsform enthält die Zusammensetzung keine Inhaltsstoffe tierischer Herkunft. Mit anderen Worten, die Zusammensetzung eignet sich für einen vegetarischen und/oder veganen Lebensstil.

Aus den Bestrebungen, die in der Einleitung genannte Aufgabe zu lösen, resultiert ferner ein Spray, umfassend eine Sprühvorrichtung und eine Speicherkammer, wobei die Speicherkammer eine erfindungsgemäße Zusammensetzung enthält. Die Sprühvorrichtung weist Mittel auf, um die erfindungsgemäße Zusammensetzung aus der Speicherkammer zu führen und in Form feiner Tröpfchen oder eines zerstäubenden Strahles an die Umgebung abzugeben. Geeignete Sprays, umfassend eine Sprühvorrichtung und eine Speicherkammer, sind dem Fachmann und dem Anwender z.B. von Mund-, Hals- und Rachensprays aus der Apotheke oder der Drogerie bekannt. Durch Betätigen, vorzugsweise manuelles Herunterdrücken, eines Auslösers auf der Sprühvorrichtung (Zerstäuber) kann der Anwender einen Sprühstoß einer Flüssigkeit mit üblicherweise wässriger oder ethanolischer Grundlage aus der Speicherkammer in Form feiner Tröpfchen oder eines zerstäubenden Strahles an die Umgebung abgeben. Ein erfindungsgemäßes Spray umfasst eine erfindungsgemäße Zusammensetzung in einer solchen handelsüblichen Vorrichtung mit Sprühvorrichtung und Speicherkammer.

Aus den Bestrebungen, die in der Einleitung genannte Aufgabe zu lösen, resultiert die Verwendung einer Sprühvorrichtung zum Verteilen einer erfindungsgemäßen Zusammensetzung.

Vorzugsweise ist das Verteilen ein Sprühen.

Vorzugsweise ist die erfindungsgemäße Verwendung eine nichtmedizinische Verwendung.

Aus den Bestrebungen, die in der Einleitung genannte Aufgabe zu lösen, resultiert ferner ein nichtmedizinisches Verfahren zur Konditionierung eines Abschnittes des menschlichen Körpers zum Erreichen mindestens eines Effektes ausgewählt aus i) Unterstützung der Erhaltung der Funktion der Schleimhaut, ii) Unterstützung der Funktion des Immunsystems, iii) Unterstützung des Schutzes von Zellen vor oxidativem Stress, iv) Verbesserung des Atemgeruches (Mundgeruches), v) Verbesserung des Geschmackes, vi) Verbesserung des Mundgefühles und vii) Unterstützung bei der Abwehr von Krankheitserregern ausgewählt aus Viren und Bakterien, wobei das Verfahren das Auftragen einer erfindungsgemäßen Zusammensetzung auf den Abschnitt des menschlichen Körpers umfasst.

Vorzugsweise ist das Auftragen ein Aufsprühen, vorzugsweise mittels Sprühvorrichtung (eines Sprays).

Der Abschnitt des menschlichen Körpers ist vorzugsweise ausgewählt aus der Mundhöhle und dem Rachen.

Nachfolgend wird die Erfindung an Hand eines Beispieles weiter veranschaulicht.

Eine beispielhafte erfindungsgemäße Zusammensetzung enthält pro 100 g der Zusammensetzung
- 33 g Wasser,
- 2 g Lecithin (1,5 g Phosphatidylcholin),
- 1,6 g Minzöl (Mentha arvensis),
- 0,4 g Gewürznelkenöl (Syzygium aromaticum),
- 0,4 g Ingwerwurzelstock-CO₂-Destraktionsextrakt (Zingiber officinale),
- 0,8 g Thymianöl (Thymus vulgaris),
- 2 g mittelkettige Triacylglycerole,
- 0,05 g (1,3)-(1,6)-Beta-D-Glucane,
- 0,16 g eines Extraktes aus Weintraubenkernen (Vitis vinifera),
- 0,2 g Vitamin B2,
- 0,007 g Vitamin B7 (Biotin),
- 0,0004 g Vitamin B12,
- 10000 IE bis 50000 IE Vitamin D3,
- 0,13 g Retinylpalmitat,
- 2 g Vitamin E-Acetat und
- Glycerin ad 100 g.

Die Zusammensetzung ist bei Raumtemperatur (25 °C) flüssig bzw. eine Flüssigkeit. Alle Inhaltstoffe kommen natürlich vor. Die Zusammensetzung enthält keine Wesentlichen Mengen an Monosaccharid, Disaccharid, ein- oder zweiwertigem Alkohol, synthetischem Tensid, künstlichem Konservierungsmittel oder künstlichem Süßstoff. Inhaltsstoffe tierischer Herkunft werden nicht benötigt. Die Zusammensetzung weist ein geringes Risiko für Unverträglichkeiten wie Allergien auf. Die Zusammensetzung weist einen guten Geschmack und Geruch auf, ist nicht kariogen und verursacht keine Blutzuckerspitzen. Die Zusammensetzung ist zuckerfrei im Sinne der Verordnung (EG) Nr. 1924/2006 des Europäischen Parlaments und des Rates vom 20. Dezember 2006 über nährwert- und gesundheitsbezogene Angaben über Lebensmittel. Mit anderen Worten, die Zusammensetzung enthält nicht mehr als 0,5 g Zucker (Monosaccharide und Disaccharide) pro 100 g der Zusammensetzung.

Zugleich ist die Zusammensetzung mikrobiologisch und physikalisch stabil. Die Zusammensetzung kann bei Verwendung in einem Spray über eine übliche Sprühvorrichtung in den menschlichen Rachen gesprüht werden und ist geeignet, ohne Bedenken geschluckt zu werden. Die spezielle Grundlage aus Glycerin, Wasser, mittelkettigen Triacylglycerolen und Lecithin in bestimmten Mengenverhältnissen ermöglicht, dass ein Anhaften am Rachen und eine lange Wechselwirkung und ein Austausch zwischen Zusammensetzung und besprühtem Körperteil erfolgt.

Durch die Vitamine wird die Gesundheit allgemein, insbesondere die Gesundheit der Zellen des Mund- Rachenraumes, gefördert. Dank der speziellen Formulierung können die wasser- und fettlöslichen Vitamine mit einem Sprühstoß direkt auf die Schleimhäute aufgetragen werden, wodurch Schleimhäute und das Immunsystem unterstützt werden.

Durch den Einsatz des Extraktes bzw. der Extrakte kann der Anteil an konservierendem Glycerin an der Zusammensetzung gesenkt werden und somit der Anteil an Wasser erhöht werden. U.a. hierdurch wird die Zusammensetzung sprühbar. Neben der konservierenden Wirkung weisen Inhaltsstoffe des Extraktes / der Extrakte antioxidative und möglicherweise antivirale und/oder antibakterielle Wirkungen auf. Außerdem verbessert die Zusammensetzung den Geschmack, den Atemgeruch (Mundgeruch) und das Mundgefühl. Der Fachmann wird anerkennen, dass die einzelnen Bestandteile der Zusammensetzung in besonderer Weise zusammenwirken, um ein das erfindungsgemäße Produkt mit all seinen vorteilhaften Eigenschaften zu bilden.

## Patentansprüche

1. Zusammensetzung, **dadurch gekennzeichnet, dass** die Zusammensetzung aus einer polaren Phase und einer unpolaren Phase besteht, wobei
die polare Phase bezogen auf das Gesamtgewicht der polaren Phase mindestens 20 Gew.-% Wasser enthält,
die Zusammensetzung pro 100 g der Zusammensetzung 0,5 g bis 4 g Lecithin enthält, und
die unpolare Phase mindestens einen Extrakt enthält, der aus einem Extrakt aus der Minze (Mentha arvensis oder Mentha x piperita), einem Extrakt aus dem Gewürznelkenbaum (Syzygium aromaticum), einem Extrakt aus dem Ingwer (Zingiber officinale) und einem Extrakt aus dem Echten Thymian (Thymus vulgaris) ausgewählt ist, wobei
die polare Phase bezogen auf das Gesamtgewicht der polaren Phase 53 Gew.-% bis 78 Gew.-% Glycerin enthält und
die unpolare Phase pro 100 g der Zusammensetzung 0,4 g bis 10,0 g des Extraktes oder der Extrakte insgesamt enthält.

2. Zusammensetzung mindestens nach Anspruch 1, **dadurch gekennzeichnet, dass** die unpolare Phase einen Extrakt aus der Minze (Mentha arvensis oder Mentha x piperita), einen Extrakt aus dem Gewürznelkenbaum (Syzygium aromaticum), einen Extrakt aus dem Ingwer (Zingiber officinale) und einen Extrakt aus dem Echten Thymian (Thymus vulgaris) enthält.

3. Zusammensetzung mindestens nach einem der Ansprüche 1 bis 2, **dadurch gekennzeichnet, dass** die unpolare Phase einen Extrakt aus der Ackerminze (Mentha arvensis), einen Extrakt aus dem Gewürznelkenbaum (Syzygium aromaticum), einen Extrakt aus dem Ingwer (Zingiber officinale) und einen Extrakt aus dem Echten Thymian (Thymus vulgaris) enthält.

4. Zusammensetzung mindestens nach einem der Ansprüche 1 bis 3, **dadurch gekennzeichnet, dass**
die polare Phase bezogen auf das Gesamtgewicht der polaren Phase 53 Gew.-% bis 67 Gew.-%, am bevorzugtesten 56 Gew.-% bis 64 Gew.-%, Glycerin enthält und
die unpolare Phase pro 100 g der Zusammensetzung 1,5 g bis 7,0 g des Extraktes oder der Extrakte insgesamt enthält.

5. Zusammensetzung mindestens nach einem der Ansprüche 1 bis 4, **dadurch gekennzeichnet, dass** die Zusammensetzung pro 100 g der Zusammensetzung 0,01 g bis 2,0 g Polysaccharide, bevorzugt β-Glucane, besonders bevorzugt (1,3)-(1,6)-Beta-D-Glucane, enthält.

6. Zusammensetzung mindestens nach einem der Ansprüche 1 bis 5, **dadurch gekennzeichnet, dass** die unpolare Phase pro 100 g der Zusammensetzung 0,1 g bis 4,0 g eines Extraktes aus der Ackerminze (Mentha arvensis) enthält.

7. Zusammensetzung mindestens nach einem der Ansprüche 1 bis 6, **dadurch gekennzeichnet, dass** die unpolare Phase pro 100 g der Zusammensetzung 0,1 g bis 2,0 g eines Extraktes aus dem Gewürznelkenbaum (Syzygium aromaticum) enthält.

8. Zusammensetzung mindestens nach einem der Ansprüche 1 bis 7, **dadurch gekennzeichnet, dass** die unpolare Phase pro 100 g der Zusammensetzung 0,1 g bis 2,0 g eines Extraktes, vorzugsweise eines CO₂-Destraktionsextraktes, aus dem Ingwer (Zingiber officinale) enthält.

9. Zusammensetzung mindestens nach einem der Ansprüche 1 bis 8, **dadurch gekennzeichnet, dass** die unpolare Phase pro 100 g der Zusammensetzung 0,1 g bis 2,0 g eines Extraktes aus dem Echten Thymian (Thymus vulgaris) enthält.

10. Zusammensetzung mindestens nach einem der Ansprüche 1 bis 9, **dadurch gekennzeichnet, dass** die Zusammensetzung pro 100 g der Zusammensetzung 0,05 g bis 2,0 g eines Extraktes aus Weintrauben (Vitis vinifera), vorzugsweise aus Weintraubenkernen, enthält.

11. Zusammensetzung mindestens nach einem der Ansprüche 1 bis 10, **dadurch gekennzeichnet, dass** die unpolare Phase pro 100 g der Zusammensetzung zusätzlich zu in dem Extrakt/den Extrakten enthaltenen Triacylglycerolen 0,5 g bis 5,0 g Triacylglycerole, vorzugsweise mittelkettige Triacylglycerole, enthält.

12. Zusammensetzung mindestens nach einem der Ansprüche 1 bis 11, **dadurch gekennzeichnet, dass** die Zusammensetzung mindestens ein Vitamin, ausgewählt aus Vitamin B2, Vitamin B7 (Biotin), Vitamin B12, Vitamin D3 sowie Derivaten davon, Vitamin A sowie Derivaten davon und Vitamin E sowie Derivaten davon, enthält.

13. Spray, umfassend eine Sprühvorrichtung und eine Speicherkammer, wobei die Speicherkammer eine Zusammensetzung mindestens nach einem der Ansprüche 1 bis 12 enthält.

14. Verwendung einer Sprühvorrichtung zum Verteilen einer Zusammensetzung mindestens nach einem der Ansprüche 1 bis 12.

15. Nichtmedizinisches Verfahren zur Konditionierung eines Abschnittes des menschlichen Körpers zum Erreichen mindestens eines Effektes ausgewählt aus i) Unterstützung der Erhaltung der Funktion der Schleimhaut, ii) Unterstützung der Funktion des Immunsystems, iii) Unterstützung des Schutzes von Zellen vor oxidativem Stress, iv) Verbesserung des Atemgeruches, v) Verbesserung des Geschmackes, vi) Verbesserung des Mundgefühles und vii) Unterstützung bei der Abwehr von Krankheitserregern ausgewählt aus Viren und Bakterien, wobei das Verfahren das Auftragen einer Zusammensetzung mindestens nach einem der Ansprüche 1 bis 12 auf den Abschnitt des menschlichen Körpers umfasst.

## Claims

1. Composition, **characterized in that** the composition consists of a polar phase and a nonpolar phase, wherein
the polar phase contains at least 20 wt.% water in relation to the total weight of the polar phase,
the composition contains 0.5 g to 4 g lecithin per 100 g of the composition, and
the nonpolar phase contains at least one extract selected from an extract of mint (Mentha arvensis or Mentha x piperita), an extract of the clove tree (Syzygium aromaticum), an extract of ginger (Zingiber officinale), and an extract of common thyme (Thymus vulgaris), wherein
the polar phase contains 53 wt.% to 78 wt.% glycerin in relation to the total weight of the polar phase and
the nonpolar phase contains 0.4 g to 10.0 g of the extract or the extracts per 100 g of the composition.

2. Composition at least as claimed in claim 1, **characterized in that** the nonpolar phase contains an extract of mint (Mentha arvensis or Mentha x piperita), an extract of the clove tree (Syzygium aromaticum), an extract of ginger (Zingiber officinale), and an extract of common thyme (Thymus vulgaris).

3. Composition as claimed in at least one of claims 1 to 2, **characterized in that** the nonpolar phase contains an extract of field mint (Mentha arvensis), an extract of the clove tree (Syzygium aromaticum), an extract of ginger (Zingiber officinale), and an extract of common thyme (Thymus vulgaris).

4. Composition as claimed in at least one of claims 1 to 3, **characterized in that**
the polar phase contains 53 wt.% to 67 wt.%, most preferably 56 wt.% to 64 wt.%, glycerin in relation to the total weight of the polar phase and
the nonpolar phase contains 1.5 g to 7.0 g of the extract or the extracts in total per 100 g of the composition.

5. Composition as claimed in at least one of claims 1 to 4, **characterized in that** the composition contains 0.1 g to 2.0 g polysaccharides, preferably β-glucans, particularly preferably (1,3)-(1,6)-beta-D-glucans, per 100 g of the composition.

6. Composition as claimed in at least one of claims 1 to 5, **characterized in that** the nonpolar phase contains 0.1 g to 4.0 g of an extract of field mint (Mentha arvensis) per 100 g of the composition.

7. Composition as claimed in at least one of claims 1 to 6, **characterized in that** the nonpolar phase contains 0.1 g to 2.0 g of an extract of the clove tree (Syzygium aromaticum) per 100 g of the composition.

8. Composition as claimed in at least one of claims 1 to 7, **characterized in that** the nonpolar phase contains 0.1 g to 2.0 g of an extract, preferably a CO₂ destraction extract, of ginger (Zingiber officinale) per 100 g of the composition.

9. Composition as claimed in at least one of claims 1 to 8, **characterized in that** the nonpolar phase contains 0.1 g to 2.0 g of an extract of common thyme (Thymus vulgaris) per 100 g of the composition.

10. Composition as claimed in at least one of claims 1 to 9, **characterized in that** the composition contains 0.05 g to 2.0 g of an extract of grapes (Vitis vinifera), preferably of grape seeds, per 100 g of the composition.

11. Composition as claimed in at least one of claims 1 to 10, **characterized in that** the nonpolar phase contains, in addition to triacylglycerols contained in the extract/the extracts, 0.5 g to 5.0 g triacylglycerols, preferably medium-chain triacylglycerols, per 100 g of the composition.

12. Composition as claimed in at least one of claims 1 to 11, **characterized in that** the composition contains at least one vitamin, selected from vitamin B2, vitamin B7 (biotin), vitamin B12, vitamin D3 and derivatives thereof, vitamin A and derivatives thereof, and vitamin E and derivatives thereof.

13. Spray, comprising a spray device and a storage chamber, wherein the storage chamber contains a composition as claimed in at least one of claims 1 to 12.

14. Use of a spray device for distributing a composition as claimed in at least one of claims 1 to 12.

15. Nonmedical method for conditioning a section of the human body to achieve at least one effect selected from i) assisting the maintenance of the function of the mucous membrane, ii) assisting the function of the immune system, iii) assisting the protection of cells from oxidative stress, iv) improving the breath odor, v) improving the sense of taste, vi) improving the mouth feeling, and vii) assisting in the defense from pathogens selected from viruses and bacteria, wherein the method comprises the application of a composition as claimed in at least one of claims 1 to 12 to the section of the human body.

## Revendications

1. Composition, **caractérisée en ce qu'**elle se compose d'une phase polaire et d'une phase apolaire, la phase polaire contenant au moins 20 % en poids d'eau par rapport à son poids total,
la composition contenant entre 0,5 g et 4 g de lécithine pour 100 g de la composition et
la phase apolaire contenant au moins un extrait choisi parmi un extrait de menthe (*Mentha arvensis* ou *Mentha x piperita*), un extrait de giroflier (*Syzygium aromaticum*), un extrait de gingembre (*Zingiber officinale*) et un extrait de thym commun (*Thymus vulgaris*),
la phase polaire contenant entre 53 % en poids et 78 % en poids de glycérine par rapport à son poids total et
la phase apolaire contenant entre 0,4 g et 10,0 g de l'extrait ou du total des extraits pour 100 g de la composition.

2. Composition au moins selon la revendication 1, **caractérisée en ce que** la phase apolaire contient un extrait de menthe (*Mentha arvensis* ou *Mentha x piperita*), un extrait de giroflier (*Syzygium aromaticum*), un extrait de gingembre (*Zingiber officinale*) et un extrait de thym commun (*Thymus vulgaris*)*.*

3. Composition au moins selon une des revendications 1 à 2, **caractérisée en ce que** la phase apolaire contient un extrait de menthe des champs (*Mentha arvensis*), un extrait de giroflier (*Syzygium aromaticum*), un extrait de gingembre (*Zingiber officinale*) et un extrait de thym commun (*Thymus vulgaris*)*.*

4. Composition au moins selon une des revendications 1 à 3, **caractérisée en ce que**
la phase polaire contient entre 53 % en poids et 67 % en poids, de préférence entre 56 % en poids et 64 % en poids, de glycérine par rapport à son poids total et
la phase apolaire contient entre 1,5 g et 7,0 g de l'extrait ou du total des extraits pour 100 g de la composition.

5. Composition au moins selon une des revendications 1 à 4, **caractérisée en ce qu'**elle contient entre 0,01 g et 2,0 g de polysaccharides, de préférence de β-glucanes, en particulier de (1,3)-(1,6)- bêta-D-glucanes, pour 100 g de la composition.

6. Composition au moins selon une des revendications 1 à 5, **caractérisée en ce que** la phase apolaire contient entre 0,1 g et 4,0 g d'un extrait de menthe des champs (*Mentha arvensis*) pour 100 g de la composition.

7. Composition au moins selon une des revendications 1 à 6, **caractérisée en ce que** la phase apolaire contient entre 0,1 g et 2,0 g d'une extrait de giroflier (*Syzygium aromaticum*) pour 100 g de la composition .

8. Composition au moins selon une des revendications 1 à 7, **caractérisée en ce que** la phase apolaire contient entre 0,1 g et 2,0 g d'un extrait de gingembre (*Zingiber officinale*), de préférence un extrait obtenu par déstraction au CO₂, pour 100 g de la composition .

9. Composition au moins selon une des revendications 1 à 8, **caractérisée en ce que** la phase apolaire contient entre 0,1 g et 2,0 g d'un extrait de thym commun (*Thymus vulgaris*) pour 100 g de la composition .

10. Composition au moins selon une des revendications 1 à 9, **caractérisée en ce qu'**elle contient entre 0,05 g et 2,0 g d'un extrait de raisin (*Vitis vinifera*), de préférence de pépins de raisin, pour 100 g de la composition .

11. Composition au moins selon une des revendications 1 à 10, **caractérisée en ce que** la phase apolaire contient, en plus des triacylglycérols contenus dans l'extrait ou les extraits, entre 0,5 g et 5,0 g de triacylglycérols, de préférence des triacylglycérols à chaîne moyenne, pour 100 g de la composition.

12. Composition au moins selon une des revendications 1 à 11, **caractérisée en ce qu'**elle contient au moins une vitamine choisie parmi la vitamine B2, la vitamine B7 (biotine), la vitamine B12, la vitamine D3 et ses dérivés, la vitamine A et ses dérivés et la vitamine E et ses dérivés.

13. Spray comprenant un dispositif nébuliseur et un réservoir, dans lequel le réservoir contient une composition au moins selon une des revendications 1 à 12.

14. Utilisation d'un dispositif nébuliseur pour distribuer une composition au moins selon une des revendications 1 à 12.

15. Procédé non médical pour le conditionnement d'une partie du corps humain afin d'obtenir au moins un effet parmi i) le soutien du maintien de la fonction de la muqueuse, ii) le soutien de la fonction du système immunitaire, iii) le soutien de la protection de cellules contre le stress oxydatif, iv) l'amélioration de l'haleine, v) l'amélioration du goût, vi) l'amélioration de la sensation en bouche et vii) le soutien de la défense contre des agents pathogènes choisis parmi des virus et des bactéries, lequel procédé comprend l'application d'une composition au moins selon une des revendications 1 à 12 sur la partie du corps humain.
